# EUROPÄISCHE PATENTSCHRIFT

(11) **EP 3 013 783 B1**
(45) Veröffentlichungstag und Bekanntmachung des Hinweises auf die Patenterteilung: **17.10.2018**
(21) Anmeldenummer: 14817018.6
(22) Anmeldetag: 16.06.2014
(51) Int. Cl.: C07C 51/265, C07C 51/31, C07D 307/89, C07C 63/16

(54) **VERFAHREN ZUR HERSTELLUNG VON PHTHALSÄUREANHYDRID**
PROCESS FOR PREPARING PHTHALIC ANHYDRIDE
PROCÉDÉ DE PRODUCTION D'ANHYDRIDE PHTALIQUE

(30) Priorität: 26.06.2013 EP 13173693
(43) Veröffentlichungstag der Anmeldung: 04.05.2016
(73) Patentinhaber: BASF SE, 67056 Ludwigshafen am Rhein (DE)
(72) Erfinder: FISCHER, Nico Frederik, 69115 Heidelberg (DE); KRÄMER, Michael, 67734 Katzweiler (DE); ZÜHLKE, Jürgen, 67346 Speyer (DE); ALLMANN, Hans-Martin, 74867 Neunkirchen (DE)
(74) Vertreter: BASF IP Association
(86) Internationale Anmeldenummer: PCT/IB2014/062255
(87) Internationale Veröffentlichungsnummer: WO 2014/207603

(56) Entgegenhaltungen:
- EP-A1- 0 061 018
- WO-A1-02/16299
- WO-A1-03/070680
- WO-A1-2005/042459
- DE-A1- 1 958 776
- DE-A1- 2 340 047
- GB-A- 1 281 631

## Beschreibung

Die vorliegende Erfindung betrifft ein Verfahren zur Herstellung von Phthalsäureanhydrid durch Gasphasenoxidation von aromatischen Kohlenwasserstoffen, bei dem man einen Gasstrom, der mindestens einen aromatischen Kohlenwasserstoff und molekularen Sauerstoff umfasst, kontinuierlich über einen thermostatisierten Katalysator leitet und nach Inbetriebnahme des Katalysators die Zufuhr des mindestens einen aromatischen Kohlenwasserstoffs zum Katalysator vorübergehend unterbricht.

Eine Vielzahl von Carbonsäuren und/oder Carbonsäureanhydriden wird technisch durch die katalytische Gasphasenoxidation von Kohlenwasserstoffen wie Benzol, den Xylolen, Naphthalin, Toluol oder Durol in Festbettreaktoren hergestellt. Man kann auf diese Weise z. B. Benzoesäure, Maleinsäureanhydrid, Phthalsäureanhydrid (PSA), Isophthalsäure, Terephthalsäure oder Pyromellithsäureanhydrid erhalten. Beispielsweise wird Phthalsäureanhydrid technisch durch katalytische Gasphasenoxidation von o-Xylol und/oder Naphthalin hergestellt. Ausgangsmaterial ist ein Gemisch aus einem molekularen Sauerstoff enthaltenden Gas, beispielsweise Luft, und dem zu oxidierenden o-Xylol und/oder Naphthalin. Das Gemisch wird durch eine Vielzahl in einem Reaktor angeordneter Rohre (Rohrbündelreaktor) geleitet, in denen sich eine Schüttung mindestens eines Katalysators befindet. Zur Temperaturregelung und Thermostatisierung sind die Rohre von einem Wärmeträgermedium, beispielsweise einer Salzschmelze, umgeben.

Die katalytische Gasphasenoxidation von o-Xylol und/oder Naphthalin zu PSA ist allgemein bekannt und beispielsweise in WO 2004/103561 beschrieben. Da die Umsetzung stark exotherm ist, kommt es innerhalb des Reaktors zur Ausbildung von Temperaturmaxima (sog. hot spots), welche üblicherweise im Temperaturbereich von 400 bis 500 °C, insbesondere im Temperaturbereich von 410 bis 460 °C liegen. Hot spot-Temperaturen über 500 °C führen zu einer starken Abnahme der erreichbaren PSA-Ausbeute sowie der Katalysatorstandzeit. Zu niedrige hot spot-Temperaturen führen dagegen zu einem zu großen Gehalt an Unteroxidationsprodukten im Phthalsäureanhydrid (insbesondere Phthalid bzw. Naphthochinon), wodurch die Produktqualität entscheidend beeinträchtigt wird. Die hot spot-Temperatur hängt u. a. von der Eduktbeladung des Gasstroms, von der Höhe der Katalysatorbelastung, vom Alterungszustand des Katalysators, von den charakteristischen Wärmeübergangsverhältnissen des Reaktors (welche u. a von den Reaktorrohrdimensionen und dem Salzbadvolumen abhängen) und von der Temperatur des Wärmeträgermediums ab.

Das Einstellen und Konstanthalten der Temperatur ist somit für die Leistungsfähigkeit des Verfahrens von großer Bedeutung, die sich in erster Linie an der erhaltenen PSA-Ausbeute sowie an dessen Reinheit bemisst. Qualitativ hochwertiges PSA (vgl. DE 10 2010 006 854 A1, WO 2011/128814 A1) zeichnet sich durch möglichst niedrige Gehalte sowohl an Unteroxidationsprodukten (insbesondere Phthalid bzw.Naphthochinon) als auch an nicht umgesetztem o-Xylol bzw. Naphthalin aus, da diese Stoffe sich nur sehr schwer voneinander trennen lassen und die Farbzahl des fertigen rein-PSA negativ beeinflussen.

Der Einfluss von An- und Abfahrprozessen auf die Stabilität von Katalysatoren für die Oxidation von o-Xylol zu PSA im Modellversuch wurde untersucht und von H. Bo et al. in Petrochemical Technology 2004, Bd. 5, Seiten 421 bis 423 beschrieben.

DE-A-1958776 beschreibt ein Verfahren zur Herstellung von Phthalsäureanhydrid, bei dem man die volle Beladung des Katalysators erst nach Ausbildung eines hot spots einstellt. GB-A-1,281,631 offenbart eine Methode zur Steuerung eines PSA-Reaktors, welche es erlaubt die Temperatur des Wärmeträgermediums in Anhängigkeit von der Produktgaszusammensetzung anzupassen. EP 0 061 018 A1 beschreibt ein Verfahren zur Reaktivierung von Vanadium enthaltenden Oxidationskatalysatoren, u.a. durch Behandlung mit Schwefeltrioxid. DE-A-2340047 offenbart ein Verfahren zum Entfernen von Ablagerungen von Katalysatoren durch schlagartiges Entspannen eines im Reaktor aufgebauten Überdrucks.

Es besteht ein ständiger Bedarf nach verbesserten Verfahren für Gasphasenoxidationen, die einen möglichst hohen Umsatz bei hoher Selektivität und Produktreinheit ermöglichen.

Der vorliegenden Erfindung lag die Aufgabe zugrunde, ein Verfahren zur Herstellung von Phthalsäureanhydrid durch Gasphasenoxidation von aromatischen Kohlenwasserstoffen bereitzustellen, welches ein möglichst reines Produkt bei hohem Umsatz und gleichzeitig hoher Selektivität bietet.

Diese Aufgabe wird gelöst durch ein Verfahren zur Herstellung von Phthalsäureanhydrid durch Gasphasenoxidation von aromatischen Kohlenwasserstoffen, bei dem man einen Gasstrom, der mindestens einen aromatischen Kohlenwasserstoff und molekularen Sauerstoff umfasst, kontinuierlich über einen thermostatisierten Katalysator leitet und nach Inbetriebnahme des Katalysators die Zufuhr des mindestens einen aromatischen Kohlenwasserstoffs zum Katalysator vorübergehend unterbricht.

Gegenstand der Erfindung ist somit ein Verfahren zur Herstellung von Phthalsäureanhydrid durch Gasphasenoxidation von aromatischen Kohlenwasserstoffen, bei dem man einen Gasstrom, der mindestens einen aromatischen Kohlenwasserstoff und molekularen Sauerstoff umfasst, kontinuierlich über einen thermostatisierten Katalysator leitet, dadurch gekennzeichnet, dass man nach Inbetriebnahme des Katalysators die Zufuhr des mindestens einen aromatischen Kohlenwasserstoffs zum Katalysator für einen Zeitraum unterbricht und am Ende des Zeitraums die Zufuhr des mindestens einen aromatischen Kohlenwasserstoffs zum Katalysator wieder aufnimmt und die Temperatur des Wärmeträgermediums auf einen höheren Wert einstellt als vor der Unterbrechung der Zufuhr des aromatischen Kohlenwasserstoffs zum Katalysator.

Als Katalysatoren haben sich für diese Oxidationsreaktionen sogenannte Schalenkatalysatoren bewährt, bei denen die katalytisch aktive Masse schalenförmig auf einem inerten Trägermaterial, wie Steatit, aufgebracht ist. Als katalytisch aktive Bestandteile der katalytisch aktiven Masse dieser Schalenkatalysatoren werden im Allgemeinen Titandioxid und Vanadiumpentoxid verwendet. Des Weiteren können in der katalytisch aktiven Masse in geringen Mengen eine Vielzahl anderer oxidischer Verbindungen enthalten sein, die als Promotoren die Aktivität und Selektivität des Katalysators beeinflussen.

Als inertes Trägermaterial können praktisch alle Trägermaterialien des Standes der Technik, wie sie vorteilhaft bei der Herstellung von Schalenkatalysatoren für die Oxidation von aromatischer Kohlenwasserstoffe zu Aldehyden, Carbonsäuren und /oder Carbonsäureanhydriden eingesetzt werden, Verwendung finden, wie beispielsweise Quarz (SiO₂), Porzellan, Magnesiumoxid, Zinndioxid, Siliciumcarbid, Rutil, Tonerde (Al₂O₃), Aluminiumsilikat, Steatit (Magnesiumsilikat), Zirkoniumsilikat, Cersilikat oder Mischungen dieser Trägermaterialien. Die Katalysatorträger können beispielsweise in Form von Kugeln, Ringen, Tabletten, Spiralen, Röhren, Extrudaten oder Splitt verwendet werden. Die Dimensionen dieser Katalysatorträger entsprechen denen üblicherweise zur Herstellung von Schalenkatalysatoren für die Gasphasenreaktionen von aromatischen Kohlenwasserstoffen verwendeten Katalysatorträgern. Bevorzugt wird Steatit in Form von Kugeln mit einem Durchmesser von 3 bis 6 mm oder von Ringen mit einem äußeren Durchmesser von 5 bis 9 mm und einer Länge von 3 bis 8 mm und einer Wandstärke von 1 bis 2 mm verwendet.

Für die katalytische Gasphasenoxidation von o-Xylol und/oder Naphthalin zu PSA geeignete Katalysatoren enthalten eine katalytisch aktive Masse, welche mindestens Vanadiumoxid und Titandioxid umfasst und in einer oder mehreren Schichten auf das Trägermaterial aufgebracht werden kann. Verschiedene Schichten können sich dabei in ihrer Zusammensetzung unterscheiden.

Vorzugsweise enthält die katalytisch aktive Masse, bezogen auf die Gesamtmenge der katalytisch aktiven Masse, 1 bis 40 Gew.-% Vanadiumoxid, berechnet als V₂O₅, und 60 bis 99 Gew.-% Titandoxid, berechnet als TiO₂. Die katalytisch aktive Masse kann in bevorzugten Ausführungsformen daneben bis zu 1 Gew.-% einer Cäsiumverbindung, berechnet als Cs, bis zu 1 Gew.-% einer Phosphorverbindung, berechnet als P, und bis zu 10 Gew.-% Antimonoxid, berechnet als Sb₂O₃ enthalten. Alle Angaben zur Zusammensetzung der katalytisch aktiven Masse beziehen sich auf deren kalzinierten Zustand, z. B. nach Kalzinierung des Katalysators für eine Stunde bei 450 °C.

Üblicherweise wird Titandioxid in der Anatas-Form für katalytisch aktive Masse verwendet. Das Titandioxid weist vorzugsweise eine BET-Oberfläche von 15 bis 60 m²/g, insbesondere 15 bis 45 m²/g, besonders bevorzugt 13 bis 28 m²/g auf. Das eingesetzte Titandioxid kann aus einem einzelnen Titandioxid oder einem Gemisch von Titandioxiden bestehen. In letzterem Fall bestimmt sich der Wert der BET-Oberfläche als gewichteter Mittelwert der Beiträge der einzelnen Titandioxide. Das eingesetzte Titandioxid besteht z. B. vorteilhaft aus einem Gemisch eines TiO₂ mit einer BET-Oberfläche von 5 bis 15 m²/g und eines TiO₂ mit einer BET-Oberfläche von 15 bis 50 m²/g.

Als Vanadiumquelle eignen sich besonders Vanadiumpentoxid oder Ammoniummetavanadat. Als Antimonquelle eignen sich verschiedene Antimonoxide, insbesondere Antimontrioxid. Vanadium und Antimon können darüber hinaus auch in Form einer Vanadiumantimonatverbindung eingesetzt werden (WO 2011/061132). Das in der Aktivmasse mindestens einer Lage eingebrachte Vanadiumantimonat kann durch Umsetzung beliebiger Vanadium- und Antimonverbindungen hergestellt werden. Bevorzugt ist die direkte Umsetzung der Oxide zu einem Mischoxid bzw. Vanadiumantimonat. Das Vanadiumantimonat kann unterschiedliche molare Verhältnisse von Vanadium zu Antimon aufweisen sowie ggf. auch weitere Vanadium- bzw. Antimonverbindungen enthalten und in Mischung mit weiteren Vanadium- bzw. Antimonverbindungen eingesetzt werden.

Als Phosphorquelle kommen insbesondere Phosphorsäure, phosphorige Säure, hypophosphorige Säure, Ammoniumphosphat oder Phosphorsäureester und vor allem Ammoniumdihydrogenphosphat in Betracht. Als Quellen von Cäsium kommen das Oxide oder Hydroxid oder die thermisch in das Oxid überführbare Salze wie Carboxylate, insbesondere das Acetat, Malonat oder Oxalat, Carbonat, Hydrogencarbonat, Sulfat oder Nitrat in Betracht.

Neben den optionalen Zusätzen Cäsium und Phosphor können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Als solche Promotoren seien beispielhaft die Alkalimetalle, insbesondere außer dem genannten Cäsium, Lithium, Kalium und Rubidium, die meist in Form ihrer Oxide oder Hydroxide eingesetzt werden, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid genannt.

Ferner kommen von den genannten Promotoren noch bevorzugt als Zusätze die Oxide von Niob und Wolfram in Mengen von 0,01 bis 0,50 Gew.-%, bezogen auf die katalytisch wirksame Masse in Betracht.

Das Aufbringen der Schicht(en) des Schalenkatalysators erfolgt zweckmäßigerweise durch Aufsprühen einer Suspension von TiO₂ und V₂O₅, die gegebenenfalls Quellen der oben genannten Promotorelemente enthält, auf den fluidisierten Träger (EP 1670741, WO 2005/030388). Vor der Beschichtung wird die Suspension vorzugsweise ausreichend lange, z. B. 2 bis 30 Stunden, insbesondere 12 bis 25 Stunden, gerührt, um Agglomerate der suspendierten Feststoffe aufzubrechen und eine homogene Suspension zu erhalten. Die Suspension hat typischerweise einen Feststoffgehalt von 20 bis 50 Gew.-%. Das Suspensionsmedium ist im Allgemeinen wässrig, z. B. Wasser selbst oder ein wässriges Gemisch mit einem wassermischbaren organischen Lösungsmittel, wie Methanol, Ethanol, Isopropanol, Formamid und dergleichen.

In der Regel werden der Suspension organische Binder, bevorzugt Copolymere, vorteilhaft in Form einer wässrigen Dispersion, von Acrylsäure/Maleinsäure, Vinylacetat/Vinyllaurat, Vinylacetat/Acrylat, Styrol/Acrylat sowie Vinylacetat/Ethylen zugesetzt. Die Binder sind als wässrige Dispersionen handelsüblich, mit einem Feststoffgehalt von z. B. 35 bis 65 Gew.-%. Die eingesetzte Menge solcher Binderdispersionen beträgt im Allgemeinen 2 bis 45 Gew.-%, vorzugsweise 5 bis 35 Gew.-%, besonders bevorzugt 7 bis 20 Gew.-%, bezogen auf das Gewicht der Suspension.

Der Träger wird in z. B. einer Wirbelschicht- bzw. Fließbettapparatur in einem aufsteigenden Gasstrom, insbesondere Luft, fluidisiert. Die Apparate bestehen meist aus einem konischen oder kugelförmigen Behälter, bei dem das fluidisierende Gas von unten oder von oben über ein Tauchrohr eingeführt wird. Die Suspension wird über Düsen von oben, seitlich oder von unten in die Wirbelschicht eingesprüht. Vorteilhaft ist der Einsatz eines mittig bzw. konzentrisch um das Tauchrohr angeordneten Steigrohrs. Innerhalb des Steigrohres herrscht eine höhere Gasgeschwindigkeit, die die Trägerpartikel nach oben transportiert. Im äußeren Ring liegt die Gasgeschwindigkeit nur wenig oberhalb der Lockerungsgeschwindigkeit. So werden die Partikel kreisförmig vertikal bewegt. Eine geeignete Fließbettvorrichtung ist z. B. in der DE-A 4006935 beschrieben.

Bei der Beschichtung des Katalysatorträgers mit der katalytisch aktiven Masse werden im Allgemeinen Beschichtungstemperaturen von 20 bis 500 °C angewandt, wobei die Beschichtung unter Atmosphärendruck oder unter reduziertem Druck erfolgen kann. Im Allgemeinen erfolgt die Beschichtung bei 0 °C bis 200 °C, vorzugsweise bei 20 bis 150 °C, insbesondere bei 60 bis 120 °C durchgeführt.

Die Schichtdicke der katalytisch aktiven Masse beträgt in der Regel 0,02 bis 0,2 mm, vorzugsweise 0,05 bis 0,15 mm. Der Aktivmasseanteil am Katalysator beträgt üblicherweise 5 bis 25 Gew.-%, meist 7 bis 15 Gew.-%.

Durch thermische Behandlung des so erhaltenen Präkatalysators bei Temperaturen über 200 bis 500 °C entweicht das Bindemittel durch thermische Zersetzung und/oder Verbrennung aus der aufgetragenen Schicht. Vorzugsweise erfolgt die thermische Behandlung in situ im Gasphasenoxidationsreaktor.

Es hat sich als besonders vorteilhaft erwiesen, wenn eine Katalysatorschüttung aus verschiedenen Katalysatoren eingesetzt wird, die sich in ihrer katalytischen Aktivität und/oder der chemischen Zusammensetzung ihrer Aktivmasse unterscheiden und die nacheinander als Lagen in unterschiedliche Zonen des Reaktors eingefüllt werden. Vorzugsweise wird bei Anwendung zweier Reaktionszonen in der ersten, also zum Eintritt des Gasstroms hin gelegenen Reaktionszone, ein Katalysator eingesetzt, der in Vergleich zum Katalysator, welcher sich in der zweiten, also zum Austritt des Gasstroms hin gelegenen Reaktionszone, befindet, eine etwas geringere katalytische Aktivität aufweist. Im Allgemeinen wird die Umsetzung durch die Temperatureinstellung so gesteuert, dass in der ersten Zone der größte Teil der im Gasstrom enthaltenen aromatischen Kohlenwasserstoffe bei maximaler Ausbeute umgesetzt wird. Bevorzugt werden dreibis fünflagige Katalysatorsysteme verwendet, insbesondere drei- und vierlagige Katalysatorsysteme. Ein dreilagiges Katalysatorsystem für die o-Xyloloxidation zu PSA ist beispielsweise in EP 1084115 beschrieben.

Anstelle gegeneinander abgegrenzter Lagen der verschiedenen Katalysatoren kann auch ein quasi-kontinuierlicher Übergang der Lagen und damit eine quasi gleichmäßige Änderung der Aktivmassezusammensetzung bzw. ihres Gehalts dadurch bewirkt werden, dass man beim Übergang von einer Lage zur nächsten Lage eine Zone mit einer Vermischung der aufeinander folgenden Katalysatoren einfügt.

Die Schüttungslänge der ersten Katalysatorlage (KL1) liegt vorzugsweise im Bereich von 10 bis 50 %, besonders bevorzugt im Bereich von 15 bis 30 % der gesamten Katalysatorfüllhöhe im Reaktor. Typische Reaktoren weisen eine Füllhöhe von 250 cm bis 400 cm auf. Die Katalysatorlagen können gegebenenfalls auch auf mehrere Reaktoren verteilt werden.

Beim erfindungsgemäßen Verfahren wird ein Gasstrom, der mindestens einen aromatischen Kohlenwasserstoff und molekularen Sauerstoff umfasst, kontinuierlich über einen solchen thermostatisierten Katalysator geleitet. Der molekulare Sauerstoff wird üblicherweise der Luft entnommen. Als aromatische Kohlenwasserstoffe kommen bevorzugt die Xylole oder Naphthalin sowie deren Gemische zum Einsatz, besonders bevorzugt o-Xylol oder Naphthalin sowie deren Gemische. Die katalytische Gasphasenoxidation von o-Xylol und/oder Naphthalin zu PSA wird häufig in einem Rohrbündelreaktor durchgeführt, in dessen Rohren sich der Katalysator in Form einer Schüttung befindet. Über diese Katalysatorschüttung wird ein Gasstrom, der mindestens einen aromatischen Kohlenwasserstoff und molekularen Sauerstoff umfasst, bei Temperaturen von im Allgemeinen 300 bis 450 °C, vorzugsweise 320 bis 420 °C und besonders bevorzugt 340 bis 400 °C, und bei einem Überdruck von im Allgemeinen 0,1 bis 2,5 bar, vorzugsweise 0,3 bis 1,5 bar, und mit einer Raumgeschwindigkeit von im Allgemeinen 750 bis 5000 h⁻¹, vorzugsweise 2000 bis 5000 h⁻¹, geleitet. Der dem Katalysator zugeführte Gasstrom (Eduktgas) wird im Allgemeinen durch Vermischen eines molekularen Sauerstoff enthaltenden Gases, das außer Sauerstoff noch geeignete Reaktionsmoderatoren und/oder Verdünnungsmittel, wie Dampf, Kohlendioxid und/oder Stickstoff enthalten kann, mit dem zu oxidierenden o-Xylol und/oder Naphthalin erzeugt. Der Gasstrom enthält im Allgemeinen 1 bis 100 mol%, vorzugsweise 2 bis 50 mol% und besonders bevorzugt 10 bis 30 mol% Sauerstoff. Im Allgemeinen wird dieser Gasstrom mit 5 bis 140 g/Nm³, vorzugsweise 60 bis 120 g/Nm³ und besonders bevorzugt 80 bis 120 g/Nm³ o-Xylol und/oder Naphthalin beladen. Der Gasstrom wird vor seinem Eintritt in den Reaktor auf Temperaturen von 150 bis 350 °C vorgeheizt.

Zur Kontrolle der im Reaktor während der Reaktion auftretenden Maximaltemperaturen wird der Katalysator im Reaktor thermostatisiert, etwa indem man die Reaktorrohre mit einem Wärmeträgermedium umgibt, beispielsweise einem Salzbad. Bei Verwendung eines Mehrlagenkatalysators können auch mehrere getrennte Salzbadkreisläufe Verwendung finden, mit denen die einzelnen Lagen separat und ggf. unterschiedlich temperiert werden können.

Die Standzeit derartiger Katalysatoren beträgt im Allgemeinen etwa 4 bis 5 Jahre. Die Inbetriebnahme eines neuen Katalysators muss wegen dessen hoher Anfangsaktivität recht behutsam erfolgen. Man beginnt daher meist erst mit einer relativ niedrigen Beladung des Gasstroms mit aromatischem Kohlenwasserstoff und steigert diese allmählich über mehrere Monate bis auf den gewünschten Zielwert von etwa 80 bis 120 g/Nm³.
Im Rahmen dieser Erfindung gilt ein Katalysator als in Betrieb genommen, sobald er wenigstens einmal bei einer Reaktionstemperatur von mindestens 300 °C mit einem Gasstrom, der mindestens einen aromatischen Kohlenwasserstoff und molekularen Sauerstoff umfasst, in Kontakt gebracht wurde.

In einer Ausführungsform der Erfindung wird nach Inbetriebnahme des Katalysators die Zufuhr des mindestens einen aromatischen Kohlenwasserstoffs zum Katalysator für einen Zeitraum unterbrochen. Dies kann beispielsweise dadurch geschehen, dass man dem über den Katalysator geleiteten Gasstrom keinen aromatischen Kohlenwasserstoff mehr zusetzt. Es wird dann während des erfindungsgemäßen Zeitraums ein Kohlenwasserstoff-freier Gasstrom über den Katalysator geleitet, der in seiner Zusammensetzung variieren kann. Beispielsweise können während des Zeitraums Luft oder Inertgase wie Stickstoff oder Edelgase (z. B. Argon) oder deren Gemische mit Luft über den Katalysator geleitet werden. Der Sauerstoffgehalt des Gasstroms kann auch angereichert werden und beispielsweise bis zu 50 Vol.-% betragen.

In einer weiteren Ausführungsform der Erfindung wird der über den Katalysator geleitete Gasstrom ganz abgestellt. Dabei kann der Gasstrom direkt unterbrochen oder über einen gewissen Zeitraum hinweg allmählich abgesenkt werden, bis eine vollständige Unterbrechung erreicht ist. Vorteilhafterweise wird zunächst die Zufuhr des aromatischen Kohlenwasserstoffs zum Gasstrom unterbrochen und der Reaktor mit einem wie oben beschriebenen Kohlenwasserstoff-freier Gasstrom gespült, bis sich kein Kohlenwasserstoff mehr im Reaktor befindet. Anschließend wird die Gaszufuhr zum Reaktor ganz abgestellt.

In jedem Fall wird der Katalysator während des erfindungsgemäßen Zeitraums auf seiner Reaktionstemperatur von mindestens 300 °C gehalten, beispielsweise durch Konstanthalten der Salzbadtemperatur.

Die Länge des erfindungsgemäßen Zeitraums, während dessen dem Katalysator nach seiner Inbetriebnahme kein aromatischer Kohlenwasserstoff zugeführt wird, kann sehr stark variiert werden und zwischen einigen Minuten und bis zu einem Jahr betragen. In einer bevorzugten Ausführungsform der Erfindung liegt die Länge dieses Zeitraums im Bereich von 30 Minuten bis 200 Tagen, in einer besonders bevorzugten Ausführungsform im Bereich von 1 Stunde bis 120 Tagen, in einer ganz besonders bevorzugten Ausführungsform im Bereich von 2 Stunde bis 60 Tagen.

Die erfindungsgemäße Unterbrechung der Zufuhr des aromatischen Kohlenwasserstoffs zum Katalysator kann zu einem beliebigen Zeitpunkt nach der Inbetriebnahme des Katalysators erfolgen. Um die mit dem erfindungsgemäßen Verfahren erzielbare Verbesserung der Produktqualität alsbald zu erhalten, ist eine möglichst frühzeitige Durchführung des Verfahrens vorteilhaft. Das Verfahren kann auch schon während der in der Regel mehrmonatigen Anfahrphase durchgeführt werden, also noch bevor die angestrebte Maximalbeladung des Eduktgases mit aromatischem Kohlenwasserstoff erreicht wurde.

Am Ende des Zeitraums, während dessen dem Katalysator nach seiner Inbetriebnahme erfindungsgemäß kein aromatischer Kohlenwasserstoff zugeführt wird, wird der Gasstrom gegebenenfalls wieder angestellt und wieder mit mindestens einem aromatischen Kohlenwasserstoff beaufschlagt. In einer Ausführungsform der Erfindung werden dabei dieselben Einstellungen für die Temperatur des Wärmeträgermediums, den Gasstrom und die Beladung des Gasstroms mit aromatischem Kohlenwasserstoff gewählt die vor der Unterbrechung der Zufuhr des aromatischen Kohlenwasserstoffs zum Katalysator verwendet wurden.

Erfindungsgemäß wird am Ende des Zeitraums die Temperatur des Wärmeträgermediums auf einen höheren Wert eingestellt als vor der Unterbrechung der Zufuhr des aromatischen Kohlenwasserstoffs zum Katalysator. In einer bevorzugten Ausführungsform der Erfindung wird die Temperatur des Wärmeträgermediums um 1 bis 5 °C höher eingestellt als vor der Unterbrechung der Zufuhr des aromatischen Kohlenwasserstoffs zum Katalysator, besonders bevorzugt um 1 bis 3 °C.

In einer weiteren bevorzugten Ausführungsform der Erfindung wird am Ende des erfindungsgemäßen Zeitraums die Beladung des Gasstroms mit aromatischem Kohlenwasserstoff auf einen niedrigeren Wert eingestellt als vor der Unterbrechung der Zufuhr des aromatischen Kohlenwasserstoffs zum Katalysator. In einer besonders bevorzugten Ausführungsform der Erfindung wird die Beladung des Gasstroms mit aromatischem Kohlenwasserstoff auf einen Wert eingestellt, der 5 bis 30 % niedriger ist als vor der Unterbrechung der Zufuhr des aromatischen Kohlenwasserstoffs zum Katalysator.

Falls nach Wiederaufnahme der Zufuhr des aromatischen Kohlenwasserstoffs zum Katalysator Einstellungen für die Temperatur des Wärmeträgermediums, den Gasstrom und die Beladung des Gasstroms mit aromatischem Kohlenwasserstoff gewählt werden, die von den Einstellungen, die vor der Unterbrechung der Zufuhr des aromatischen Kohlenwasserstoffs zum Katalysator verwendet wurden, abweichen, so können die Einstellungen in der Regel innerhalb weniger Tage wieder auf die Werte zurückgeführt werden, die vor der Unterbrechung der Zufuhr des aromatischen Kohlenwasserstoffs zum Katalysator verwendet worden ist.

Durch das erfindungsgemäße Verfahren wird eine deutliche Verbesserung der Produktqualität erreicht, ausgedrückt in reduzierten Gehalten an nicht umgesetztem aromatischem Kohlenwasserstoff und auch an Unteroxidationsprodukten wie Phthalid oder Naphthochinon im erhaltenen PSA.

Das erfindungsgemäße Verfahren kann auch bei katalytischen Gasphasenoxidationen zur Herstellung anderer Carbonsäuren und/oder Carbonsäureanhydride zur Verbesserung der Produktqualität eingesetzt werden.

Ein weiterer Gegenstand der Erfindung ist die Verwendung des oben beschriebenen Verfahrens zur Verbesserung der Produktqualität bei der Phthalsäureanhydridherstellung.

Die nachfolgenden Beispiele erläutern die Erfindung ohne sie zu begrenzen.

### Beispiele

### Beispiel 1 (fällt nicht unter die Ansprüche):

### Katalysatorlage 1 (KL1) (Vanadiumantimonat als V- und Sb-Quelle):

### Herstellung des Vanadiumantimonats:

2284,1 g Vanadiumpentoxid und 1462 g Antimontrioxid (Antraco ACC-BS, ca. 4 % Valentinit und 96 % Senarmontit; Sb₂O₃ ≥ 99,8 Gew.-%; As ≤ 800 Gew.-ppm, Pb ≤ 800 Gew.-ppm, Fe ≤ 30 Gew.-ppm Fe, mittlere Partikelgröße = 1,4 µm) wurden 5,6 l demineralisiertem Wasser suspendiert und die Suspension 15 Stunden unter Rückfluss gerührt. Im Anschluss wurde die Suspension auf 80°C abgekühlt und mittels Sprühtrocknung getrocknet. Die Eingangstemperatur lag dabei bei 340°C, die Austrittstemperatur bei 110°C. Das so erhaltene Sprühpulver hatte eine BET-Oberfläche von 89 m²/g und wies einen Gehalt an Vanadium von 32 Gew.-% sowie einen Gehalt an Antimon von 30 Gew.-% auf. Das Produkt wies folgende kristalline Bestandteile auf: Valentinit (ICPDS: 11-0689): ca. 3%; Senarmontit (ICPDS: 43-1071): ca. 2%; Vanadiumantimonat (ICPDS: 81-1219): ca. 95%. Die mittlere Kristallitgröße des Vanadiumantimonates betrug ca. 9 nm.

### Suspensionsansatz und Beschichtung:

2 kg Steatitringe (Magnesiumsilikat) mit Abmessungen von 7 mm x 7 mm x 4 mm wurden in einer Fließbettapparatur mit 752 g einer Suspension aus 4,4 g Cäsiumcarbonat, 413,3 g Titandioxid (Fuji TA 100 CT; Anatas, BET-Oberfläche 27 m²/g), 222,5 g Titandioxid (Fuji TA 100; Anatas, BET-Oberfläche 7 m²/g), 86,9 g Vanadiumantimonat (wie oben hergestellt), 1870,1 g demineralisiertem Wasser und 76,7 g organischem Binder (Copolymer aus Vinylacetat und Vinyllaurat in Form einer 50 Gew.-%igen wässrigen Dispersion) beschichtet.

Nach Kalzination des Katalysators für eine Stunde bei 450 °C betrug die auf die Steatitringe aufgebrachte Aktivmasse 8,4 %. Die analysierte Zusammensetzung der Aktivmasse bestand aus 7,1 % V₂O₅, 4,5 % Sb₂O₃, 0,50 % Cs, Rest TiO₂.

### Katalysatorlage 2 (KL2) (Vanadiumpentoxid und Antimontrioxid als V- bzw. Sb-Quelle):

2 kg Steatitringe (Magnesiumsilikat) mit Abmessungen von 7 mm x 7 mm x 4 mm wurden in einer Fließbettapparatur mit 920 g einer Suspension aus 3,0 g Cäsiumcarbonat, 446,9 g Titandioxid (Fuji TA 100 C; Anatas, BET-Oberfläche 20 m²/g), 133,5 g Titandioxid (Fuji TA 100; Anatas, BET-Oberfläche 7 m²/g), 45,4 g Vanadiumpentoxid, 11,6 g Antimontrioxid, 1660,1 g demineralisiertem Wasser und 104,5 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 10,0 %. Die analysierte Zusammensetzung der Aktivmasse bestand aus 7,1 % V₂O₅, 1,8 % Sb₂O₃, 0,38 % Cs, Rest TiO₂.

### Katalysatorlage 3 (KL3) (Vanadiumpentoxid und Antimontrioxid als V- bzw. Sb-Quelle):

2 kg Steatitringe (Magnesiumsilikat) mit Abmessungen von 7 mm x 7 mm x 4 mm wurden in einer Fließbettapparatur mit 750 g einer Suspension aus 2,33 g Cäsiumcarbonat, 468,7 g Titandioxid (Fuji TA 100 C; Anatas, BET-Oberfläche 20 m²/g), 76,3 g Titandioxid (Fuji TA 100; Anatas, BET-Oberfläche 7 m²/g), 48,7 g Vanadiumpentoxid, 16,7 g Antimontrioxid, 1588,0 g demineralisiertem Wasser und 85,2 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 8,4 %. Die analysierte Zusammensetzung der Aktivmasse bestand aus 7,95 % V₂O₅, 2,7 % Sb₂O₃, 0,31 % Cs, Rest TiO₂.

### Katalysatorlage 4 (KL4) (Vanadiumpentoxid und Antimontrioxid als V- bzw. Sb-Quelle):

2 kg Steatitringe (Magnesiumsilikat) mit Abmessungen von 7 mm x 7 mm x 4 mm wurden in einer Fließbettapparatur mit 760 g einer Suspension aus 1,7 g Cäsiumcarbonat, 370,1 g Titandioxid (Fuji TA 100 CT; Anatas, BET-Oberfläche 27 m²/g), 158,6 g Titandioxid (Fuji TA 100; Anatas, BET-Oberfläche 7 m²/g), 67,3 g Vanadiumpentoxid, 14,8 g Antimontrioxid, 1587,9 g demineralisiertem Wasser und 86,3 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 8,7 %. Die analysierte Zusammensetzung der Aktivmasse bestand aus 11 % V₂O₅, 2,4 % Sb₂O₃, 0,22 % Cs, Rest TiO₂.

### Katalysatorlage 5 (KL5) (Vanadiumpentoxid und Antimontrioxid als V- bzw. Sb-Quelle):

2 kg Steatitringe (Magnesiumsilikat) mit Abmessungen von 7 mm x 7 mm x 4 mm wurden in einer Fließbettapparatur mit 850 g einer Suspension aus 389,8 g Titandioxid (Fuji TA 100 CT; Anatas, BET-Oberfläche 27 m²/g), 97,5 g Titandioxid (Fuji TA 100; Anatas, BET-Oberfläche 7 m²/g), 122,4 g Vanadiumpentoxid, 1587,9 g demineralisiertem Wasser und 96,5 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 9,1 %. Die analysierte Zusammensetzung der Aktivmasse bestand aus 20 % V₂O₅, 0,38 % P, Rest TiO₂.

Die katalystische Oxidation von o-Xylol zu Phthalsäureanhydrid wurde in einem salzbadgekühlten Rohrreaktor mit einem Rohrinnendurchmesser von 25 mm durchgeführt. Von Reaktoreingang zu Reaktorausgang wurden 80 cm KL1, 60 cm KL2, 70 cm KL3, 50 cm KL4 und 60 cm KL5 in ein 3,5 m langes Eisenrohr mit der lichten Weite von 25 mm eingefüllt. Das Eisenrohr war zur Temperaturregelung von einer Salzschmelze umgeben, eine 4 mm Außendurchmesser Thermohülse mit eingebautem Zugelement diente der Katalysatortemperaturmessung.

Das Rohr wurde stündlich von oben nach unten mit 4,0 Nm³ Luft mit Beladungen an 99 bis 99,4 Gew.-%igem o-Xylol von 30 bis 100 g/Nm³ durchströmt. Nach 172 Tagen Laufzeit wurde bei einer Salzbadtemperatur von 346°C, einem Luftstrom von 4 Nm³/h und einer o-Xylolbeladung von 100 g/Nm³ die Zufuhr des Eduktgases zum Katalysator für 6 Stunden unterbrochen und durch Stickstoff (Reaktoreingangsdruck = 260 mbar) ersetzt. Nach den 6 Stunden wurde der Katalysator wieder bei den identischen Bedingungen wie vor der Abstellung mit o-Xylol beladener Luft beaufschlagt, d. h. einer Salzbadtemperatur von 346°C, einem Luftstrom von 4 Nm³/h und einer o-Xylolbeladung von 100 g/Nm³. Nach vier weiteren Tagen wurde die Produktgaszusammensetzung analysiert (siehe Tabelle 1).

**Tabelle 1:**

| | Vor der Abstellung | Nach der Abstellung |
|---|---|---|
| Tag seit Abstellung | 0 | 4 |
| Luftmenge [Nm³/h] | 4,0 | 4,0 |
| Beladung [g_{o-X}/Nm³] | 100 | 100 |
| Salzbadtemperatur [°C] | 346 | 346 |
| o-Xylol [Gew.-%] | 0,071 | 0,070 |
| Phthalid [Gew.-%] | 0,119 | 0,100 |

Es wurde gefunden, dass unter identischen Bedingungen die Produkqualität nach der Unterbrechung der Zufuhr des aromatischen Kohlenwasserstoffs zum Katalysator verbessert wurde, erkennbar an der Verringerung des Gehalts am Unteroxidationsprodukt Phthalid bei gleichbleibender o-Xylolkonzentration im Produkt.

### Beispiel 2 (erfindungsgemäß)

Ein Rohr, welches dem in Beispiel 1 beschriebenen entspricht und welches auch mit derselben Katalysatorschüttung wie in Beispiel 1 beschrieben gefüllt ist, wurde stündlich von oben nach unten mit 4,0 Nm³ Luft mit Beladungen an 99 bis 99,4 Gew.-%igem o-Xylol von 30 bis 100 g/Nm³ durchströmt. Nach 249 Tagen Laufzeit wurde bei einer Salzbadtemperatur von 351°C, einem Luftstrom von 4 Nm³/h und einer o-Xylolbeladung von 100 g/Nm³ die Zufuhr des Eduktgases zum Katalysator für 52 Tage unterbrochen und durch Stickstoff (Reaktoreingangsdruck = 260 mbar) ersetzt. Der Katalysator wurde anschließend bei einer erhöhten Salzbadtemperatur und einer verringerten o-Xylol Beladung wieder angefahren. Innerhalb von zwei Wochen wurden die Einstellungen wie vor der Abstellung erreicht und die Produktgaszusammensetzung analysiert (siehe Tabelle 2).

**Tabelle 2:**

| | Vor der Abstellung | Wiederanfahren | Nach der Abstellung |
|---|---|---|---|
| Tag seit Abstellung | 0 | 52 | 66 |
| Luftmenge [Nm³/h] | 4,0 | 4,0 | 4,0 |
| Beladung [g_{o-X}/Nm³] | 100 | 70 | 100 |
| Salzbadtemperatur [°C] | 348 | 352 | 347,7 |
| o-Xylol [Gew.-%] | 0,021 | | 0,012 |
| Phthalid [Gew.-%] | 0,054 | | 0,032 |

### Beispiel 3 (erfindungsgemäß)

Die katalystische Oxidation von o-Xylol zu Phthalsäureanhydrid wurde in einem salzbadgekühlten Rohrreaktor mit einem Rohrinnendurchmesser von 25 mm durchgeführt. Von Reaktoreingang zu Reaktorausgang wurden 90 cm KL1, 60 cm KL2, 70 cm KL3, 50 cm KL4 und 60 cm KL5 in ein 3,5 m langes Eisenrohr mit der lichten Weite von 25 mm eingefüllt. Das Eisenrohr war zur Temperaturregelung von einer Salzschmelze umgeben, eine 4 mm Außendurchmesser Thermohülse mit eingebautem Zugelement diente der Katalysatortemperaturmessung.

Das Rohr wurde stündlich von oben nach unten mit 4,0 Nm³-Luft mit Beladungen an 99 bis 99,4 Gew.-%igem o-Xylol von 30 bis 100 g/Nm³ durchströmt. Nach 50 Tagen Laufzeit wurde bei einer Salzbadtemperatur von 360°C, einem Luftstrom von 4 Nm³/h und einer o-Xylolbeladung von 83 g/Nm³ die Zufuhr des Eduktgases zum Katalysator unterbrochen. Der Reaktor wurde etwa 2 Minuten mit Luft gespült und anschließend die Gaszufuhr für 3 Tage ganz eingestellt. Der Katalysator wurde anschließend bei einer erhöhten Salzbadtemperatur, einer verringerten o-Xylol Beladung und einem verringerten Luftstrom wieder angefahren. Innerhalb von 7 Tagen wurden die Einstellungen wie vor der Abschaltung erreicht und die Nebenprodukte in der Produktgaszusammensetzung analysiert (siehe Tabelle 3).

**Tabelle 3:**

| | Vor der Abstellung | Wiederanfahren | Nach der Abstellung |
|---|---|---|---|
| Tag seit Abstellung | 0 | 3 | 10 |
| Luftmenge [Nm³/h] | 4,0 | 3,8 | 4,0 |
| Beladung [g_{o-X}/Nm³] | 83 | 67 | 83 |
| Salzbadtemperatur [°C] | 360 | 363 | 360 |
| o-Xylol [Gew.-%] | 0,011 | | 0,009 |
| Phthalid [Gew.-%] | 0,025 | | 0,019 |

### Beispiel 4 (erfindungsgemäß)

Ein Rohr, welches dem in Beispiel 3 beschriebenen entspricht und welches auch mit derselben Katalysatorschüttung wie in Beispiel 3 beschrieben gefüllt ist, wurde stündlich von oben nach unten mit 4,0 Nm³ Luft mit Beladungen an 99 bis 99,4 Gew.-%igem o-Xylol von 30 bis 100 g/Nm³ durchströmt. Nach 12 Tagen Laufzeit wurde bei einer Salzbadtemperatur von 367°C, einem Luftstrom von 4 Nm³/h und einer o-Xylolbeladung von 58 g/Nm³ die Zufuhr des Eduktgases zum Katalysator unterbrochen. Der Reaktor wurde etwa 2 Minuten mit Luft gespült und anschließend die Gaszufuhr für 12 Stunden ganz eingestellt. Der Katalysator wurde anschließend bei einer erhöhten Salzbadtemperatur, einer gleichbleibenden o-Xylol Beladung und einem verringerten Luftstrom wieder angefahren. Innerhalb von 8 Tagen wurden die Einstellungen wie vor der Abschaltung erreicht und die Nebenprodukte in der Produktgaszusammensetzung analysiert (siehe Tabelle 4).

**Tabelle 4:**

| | Vor der Abstellung | Wiederanfahren | Nach der Abstellung |
|---|---|---|---|
| Tag seit Abstellung | 0 | 0 | 8 |
| Luftmenge [Nm³/h] | 4,0 | 3,8 | 4,0 |
| Beladung [g_{o-X}/Nm³] | 58 | 58 | 58 |
| Salzbadtemperatur [°C] | 367 | 370 | 367 |
| o-Xylol [Gew.-%] | 0,093 | | 0,046 |
| Phthalid [Gew.-%] | 0,199 | | 0,107 |

### Beispiel 5 (fällt nicht unter die Ansprüche)

Ein Rohr, welches dem in Beispiel 3 beschriebenen entspricht und welches auch mit derselben Katalysatorschüttung wie in Beispiel 3 beschrieben gefüllt ist, wurde stündlich von oben nach unten mit 4,0 Nm³ Luft mit Beladungen an 99 bis 99,4 Gew.-%igem o-Xylol von 30 bis 100 g/Nm³ durchströmt. Nach 89 Tagen Laufzeit wurde bei einer Salzbadtemperatur von 367°C, einem Luftstrom von 4 Nm³/h und einer o-Xylolbeladung von 58 g/Nm³ die Zufuhr des Eduktgases zum Katalysator unterbrochen. Der Reaktor wurde etwa 2 Minuten mit Luft gespült und anschließend die Gaszufuhr für 12 Stunden ganz eingestellt. Der Katalysator wurde anschließend bei einer gleichbleibenden Salzbadtemperatur, einer verringerten o-Xylol Beladung und einem verringerten Luftstrom wieder angefahren. Innerhalb von 5 Tagen wurden die Einstellungen wie vor der Abschaltung erreicht und die Nebenprodukte in der Produktgaszusammensetzung analysiert (siehe Tabelle 5).

**Tabelle 5:**

| | Vor der Abstellung | Wiederanfahren | Nach der Abstellung |
|---|---|---|---|
| Tag seit Abstellung | 0 | 0 | 5 |
| Luftmenge [Nm³/h] | 4,0 | 3,5 | 4,0 |
| Beladung [g_{o-X}/Nm³] | 92 | 87 | 92 |
| Salzbadtemperatur [°C] | 348 | 348 | 348 |
| o-Xylol [Gew.-%] | 0,047 | | 0,037 |
| Phthalid [Gew.-%] | 0,071 | | 0,062 |

### Beispiel 6 (nicht erfindungsgemäß)

Die katalytische Oxidation von o-Xylol zu Phthalsäureanhydrid wurde in einem salzbadgekühlten technischen Rohrreaktor mit einem Rohrinnendurchmesser von 25 mm durchgeführt. Von Reaktoreingang zu Reaktorausgang wurden 81 cm KL1, 70 cm KL2, 85 cm KL3, 50 cm KL4 und 60 cm KL5 in ein 4,0 m langes Eisenrohr mit der lichten Weite von 25 mm eingefüllt. Das Eisenrohr war zur Temperaturregelung von einer Salzschmelze umgeben.

Das Rohr wurde stündlich von oben nach unten mit 3,8 Nm³ Luft mit Beladungen an 98 bis 99 Gew.-%igem o-Xylol von 30 bis 100 g/Nm³ durchströmt. Nach 213 Tagen Laufzeit wurde die Salzbadtemperatur von 345°C, ein Luftstrom von 3,8 Nm³/h und eine o-Xylolbeladung von 95,4 g/Nm³ für 9 Tage konstant gehalten und anschließend die Nebenprodukte in der Produktgaszusammensetzung analysiert (siehe Tabelle 6).

**Tabelle 6:**

| | | |
|---|---|---|
| Laufzeit [Tage] | 213 | 222 |
| Luftmenge [Nm³/h] | 3,8 | 3,8 |
| Beladung [g_{o-X}/Nm³] | 95,4 | 95,4 |
| Salzbadtemperatur [°C] | 345 | 345 |
| o-Xylol [Gew.-%] | 0,08 | 0,08 |
| Phthalid [Gew.-%] | 0,08 | 0,08 |

### Beispiel 7 (nicht erfindungsgemäß)

Ein Rohr, welches dem in Beispiel 6 beschriebenen entspricht und welches auch mit derselben Katalysatorschüttung wie in Beispiel 6 beschrieben gefüllt ist, wurde stündlich von oben nach unten mit 3,8 Nm³ Luft mit Beladungen an 98 bis 99 Gew.-%igem o-Xylol von 30 bis 100 g/Nm³ durchströmt. Nach 273 Tagen Laufzeit wurde die Salzbadtemperatur von 345°C, ein Luftstrom von 3,8 Nm³/h und eine o-Xylolbeladung von 95,9 g/Nm³ für 21 Tage konstant gehalten und die Nebenprodukte in der Produktgaszusammensetzung analysiert (siehe Tabelle 7).

**Tabelle 7:**

| | | |
|---|---|---|
| Laufzeit [Tage] | 273 | 294 |
| Luftmenge [Nm³/h] | 3,8 | 3,8 |
| Beladung [g_{o-X}/Nm³] | 95,9 | 95,9 |
| Salzbadtemperatur [°C] | 345 | 345 |
| o-Xylol [Gew.-%] | 0,06 | 0,06 |
| Phthalid [Gew.-%] | 0,06 | 0,07 |

### Beispiel 8 (fällt nicht unter die Ansprüche)

### Katalysatorlage 1 (KL1)

### Suspensionsansatz und Beschichtung:

2 kg Steatitringe (Magnesiumsilikat) in Form von Ringen mit Abmessungen von 8 mm x 6 mm x 5 mm wurden in einer Fließbettapparatur mit 855 g einer Suspension aus 6,9 g Cäsiumcarbonat, 574,6 g Titandioxid (Fuji TA 100 C; Anatas, BET-Oberfläche 20 m²/g), 30,6 g Vanadiumpentoxid, 1.75 g Niobpentoxid, 1588.0 g demineralisiertem Wasser und 97.1 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 8,9 %. Die analysierte Zusammensetzung der Aktivmasse bestand aus 5 % V₂O₅, 0.2 % Nb₂O₅, 0,92 % Cs, Rest TiO₂.

### Katalysatorlage 2 (KL2):

2 kg Steatitringe (Magnesiumsilikat) in Form von Ringen mit Abmessungen von 8 mm x 6 mm x 5 mm wurden in einer Fließbettapparatur mit 960 g einer Suspension aus 5,0 g Cäsiumsulfat, 394,4 g Titandioxid (Fuji TA 100 CT; Anatas, BET-Oberfläche 27 m²/g), 169,5 g Titandioxid (Fuji TA 100; Anatas, BET-Oberfläche 7 m²/g), 43,3 g Vanadiumpentoxid, 1.75 g Niobpentoxid, 0,6 g Kaliumsulfat, 0.7 g Ammoniumdihydrogenphosphat, 1584.8 g demineralisiertem Wasser und 109 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 9,0 %. Die analysierte Zusammensetzung der Aktivmasse bestand aus 7 % V₂O₅, 0.2 % Nb₂O₅, 0,6 % Cs, 0,04 % K, 0.03 % P Rest TiO₂.

### Katalysatorlage 3 (KL3):

2 kg Steatitringe (Magnesiumsilikat) in Form von Ringen mit Abmessungen von 8 mm x 6 mm x 5 mm wurden in einer Fließbettapparatur mit 950 g einer Suspension aus 3,5 g Cäsiumsulfat, 395,6 g Titandioxid (Fuji TA 100 CT; Anatas, BET-Oberfläche 27 m²/g), 169,5 g Titandioxid (Fuji TA 100; Anatas, BET-Oberfläche 7 m²/g), 42,9 g Vanadiumpentoxid, 1,75 g Niobpentoxid, 0,6 g Kaliumsulfat, 0.7 g Ammoniumdihydrogenphosphat, 1585.6 g demineralisiertem Wasser und 107,9 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 9,0 %. Die analysierte Zusammensetzung der Aktivmasse bestand aus 7 % V₂O₅, 0.2 % Nb₂O₅, 0,35 % Cs, 0,04 % K, 0.03 % PRest TiO₂.

### Katalysatorlage 4 (KL4):

2 kg Steatitringe (Magnesiumsilikat) in Form von Ringen mit Abmessungen von 8 mm x 6 mm x 5 mm wurden in einer Fließbettapparatur mit 780 g einer Suspension aus 442,78 g Titandioxid (Fuji TA 100 C; Anatas, BET-Oberfläche 20 m²/g), 111,3 g Vanadiumpentoxid, 1,7 g Wolframtrioxid, 3,8 g Ammoniumdihydrogenphosphat, 1443,6 g demineralisiertem Wasser und 88,6 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 8,0 %. Die analysierte Zusammensetzung der Aktivmasse bestand aus 20 % V₂O₅, 0.18 % P, 0,24 % W Rest TiO₂.

Die katalystische Oxidation von o-Xylol/Naphthalin zu Phthalsäureanhydrid wurde in einem salzbadgekühlten Rohrreaktor mit einem Rohrinnendurchmesser von 25 mm durchgeführt. Von Reaktoreingang zu Reaktorausgang wurden 80 cm KL1, 80 cm KL2, 90 cm KL3 und 70 cm KL4 in ein 3,5 m langes Eisenrohr mit der lichten Weite von 25 mm eingefüllt. Das Eisenrohr war zur Temperaturregelung von einer Salzschmelze umgeben, eine 4 mm Außendurchmesser Thermohülse mit eingebautem Zugelement diente der Katalysatortemperaturmessung.

Das Rohr wurde stündlich von oben nach unten mit 4,0 Nm³ Luft mit Beladungen an 99 bis 99,4 Gew.-%igem o-Xylol von 0 bis 40 g/Nm³ und technischem Naphthalin von 37 bis 42 g/Nm³ durchströmt. Nach 5 Tagen Laufzeit wurde bei einer Salzbadtemperatur von 377°C, einem Luftstrom von 4 Nm³/h, einer o-Xylolbeladung von 10 g/Nm³ und einer Naphthalinbeladung von 41,2 g/Nm³ die Zufuhr des Eduktgases zum Katalysator für 2 Stunden unterbrochen und durch Stickstoff ersetzt. Nach den 2 Stunden wurde der Katalysator wieder bei den identischen Bedingungen wie vor der Abstellung mit o-Xylol und Naphthalin beladener Luft beaufschlagt, d.h. einer Salzbadtemperatur von 377°C, einem Luftstrom von 4 Nm³/h, einer o-Xylolbeladung von 10 g/Nm³ und einer Naphthalinbeladung von 41,2 g/Nm³. Nach einem weiteren Tag wurde die Produktgaszusammensetzung analysiert (siehe Tabelle 8).

**Tabelle 8:**

| | Vor der Abstellung | Nach der Abstellung |
|---|---|---|
| Tag seit Abstellung | 0 | 1 |
| Luftmenge [Nm³/h] | 4,0 | 4,0 |
| Beladung [g_{o-X}/Nm³] | 10 | 10 |
| Beladung [gₙₐₚₕₜₕₐₗᵢₙ/Nm³] | 41,2 | 41,2 |
| Salzbadtemperatur [°C] | 377 | 377 |
| o-Xylol [Gew.-%] | 0,035 | 0,008 |
| Phthalid [Gew.-%] | 0,044 | 0,013 |
| Naphthoquinone [Gew.-%] | 2,145 | 1,157 |

Es wurde gefunden, dass unter identischen Bedingungen die Produkqualität nach der Unterbrechung der Zufuhr des aromatischen Kohlenwasserstoffs zum Katalysator verbessert wurde, erkennbar an der Verringerung des Gehalts an den Unteroxidationsprodukten Phthalid und Naphthochinon bei gleichbleibender o-Xylol- und Naphthalinkonzentration im Produkt.

### Beispiel 9 (fällt nicht unter die Ansprüche)

### Katalysatorlage 1 (KL1)

### Suspensionsansatz und Beschichtung:

2 kg Steatitringe (Magnesiumsilikat) in Form von Ringen mit Abmessungen von 8 mm x 6 mm x 5 mm wurden in einer Fließbettapparatur mit 870 g einer Suspension aus 6,92 g Cäsiumcarbonat, 562,34 g Titandioxid (Fuji TA 100 C; Anatas, BET-Oberfläche 20 m²/g), 42,86 g Vanadiumpentoxid, 1.75 g Niobpentoxid, 1587.96 g demineralisiertem Wasser und 98.8 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 8,9 %. Die analysierte Zusammensetzung der Aktivmasse bestand aus 4,62 % V₂O₅, 0.28 % Nb₂O₅, 0,99 % Cs, Rest TiO₂.

### Katalysatorlage 2 (KL2):

2 kg Steatitringe (Magnesiumsilikat) in Form von Ringen mit Abmessungen von 8 mm x 6 mm x 5 mm wurden in einer Fließbettapparatur mit 970 g einer Suspension aus 5,3 g Cäsiumsulfat, 562,3 g Titandioxid (Fuji TA 100 C; Anatas, BET-Oberfläche 20 m²/g), 39,0 g Vanadiumpentoxid, 1.75 g Niobpentoxid, 1441,6 g demineralisiertem Wasser und 110,2 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 9,0 %. Die analysierte Zusammensetzung der Aktivmasse bestand aus 7 % V₂O₅, 0.2 % Nb₂O₅, 0,67 % Cs, Rest TiO₂.

### Katalysatorlage 3 (KL3):

2 kg Steatitringe (Magnesiumsilikat) in Form von Ringen mit Abmessungen von 8 mm x 6 mm x 5 mm wurden in einer Fließbettapparatur mit 900 g einer Suspension aus 3,5 g Cäsiumsulfat, 565,5 g Titandioxid (Fuji TA 100 C; Anatas, BET-Oberfläche 20 m²/g), 42,9 g Vanadiumpentoxid, 1.75 g Niobpentoxid, 1441,6 g demineralisiertem Wasser und 102,2 g organischem Binder (Copolymer aus Vinylacetat und Vinyllaurat in Form einer 50 Gew.-%igen wässrigen Dispersion) beschichtet.
Nach Kalzination des Katalysators für eine Stunde bei 450 °C betrug die auf die Steatitringe aufgebrachte Aktivmasse 9,0 %. Die analysierte Zusammensetzung der Aktivmasse bestand aus 7 % V₂O₅, 0.2 % Nb₂O₅, 0,4 % Cs, Rest TiO₂.

### Katalysatorlage 4 (KL4):

2 kg Steatitringe (Magnesiumsilikat) in Form von Ringen mit Abmessungen von 8 mm x 6 mm x 5 mm wurden in einer Fließbettapparatur mit 865 g einer Suspension aus 198,3 g Titandioxid (Fuji TA 100 CT; Anatas, BET-Oberfläche 20 m²/g), 368,3 g Titandioxid (Fuji TA 100; Anatas, BET-Oberfläche 7 m²/g), 42,9 g Vanadiumpentoxid, 1.75 g Niobpentoxid, 5,1 g Ammoniumdihydrogenphosphat, 1587,9 g demineralisiertem Wasser und 98,3 g organischem Binder (Copolymer aus Vinylacetat und Vinyllaurat in Form einer 50 Gew.-%igen wässrigen Dispersion) beschichtet. Nach Kalzination des Katalysators für eine Stunde bei 450 °C betrug die auf die Steatitringe aufgebrachte Aktivmasse 9,6 %. Die analysierte Zusammensetzung der Aktivmasse bestand aus 7 % V₂O₅, 0.2 % Nb₂O₅, 0.22 % P (in die Suspension eingebracht als Dihydrogenphosphat), Rest TiO₂.

Die katalystische Oxidation von o-Xylol/Naphthalin zu Phthalsäureanhydrid wurde in einem salzbadgekühlten Rohrreaktor mit einem Rohrinnendurchmesser von 25 mm durchgeführt. Von Reaktoreingang zu Reaktorausgang wurden 80 cm KL1, 80 cm KL2, 90 cm KL3 und 90 cm KL4 in ein 3,5 m langes Eisenrohr mit der lichten Weite von 25 mm eingefüllt. Das Eisenrohr war zur Temperaturregelung von einer Salzschmelze umgeben, eine 4 mm Außendurchmesser Thermohülse mit eingebautem Zugelement diente der Katalysatortemperaturmessung.

Das Rohr wurde stündlich von oben nach unten mit 4,0 Nm³ Luft mit Beladungen an 99 bis 99,4 Gew.-%igem o-Xylol von 0 bis 25 g/Nm³ und technischem Naphthalin von 38 bis 41 g/Nm³ durchströmt. Nach 4 Tagen Laufzeit wurde bei einer Salzbadtemperatur von 380°C, einem Luftstrom von 4 Nm³/h, einer o-Xylolbeladung von 0 g/Nm³ und einer Naphthalinbeladung von 40 g/Nm³ die Zufuhr des Eduktgases zum Katalysator für 4 Stunden unterbrochen und durch Stickstoff ersetzt. Nach den 4 Stunden wurde der Katalysator wieder bei den identischen Bedingungen wie vor der Abstellung mit Naphthalin beladener Luft beaufschlagt, d. h. einer Salzbadtemperatur von 380°C, einem Luftstrom von 4 Nm³/h, einer o-Xylolbeladung von 0 g/Nm³ und einer Naphthalinbeladung von 40 g/Nm³. Nach einem weiteren Tag wurde die Produktgaszusammensetzung analysiert (siehe Tabelle 9).

**Tabelle 9:**

| | Vor der Abstellung | Nach der Abstellung |
|---|---|---|
| Tag seit Abstellung | 0 | 1 |
| Luftmenge [Nm³/h] | 4,0 | 4,0 |
| Beladung [g_{o-X}/Nm³] | 0 | 0 |
| Beladung [gₙₐₚₕₜₕₐₗᵢₙ/Nm³] | 40 | 40 |
| Salzbadtemperatur [°C] | 380 | 380 |
| o-Xylol [Gew.-%] | 0 | 0 |
| Phthalid [Gew.-%] | 0 | 0 |
| Naphthoquinone [Gew.-%] | 1,267 | 0,516 |

Es wurde gefunden, dass unter identischen Bedingungen die Produkqualität nach der Unterbrechung der Zufuhr des aromatischen Kohlenwasserstoffs zum Katalysator verbessert wurde, erkennbar an der Verringerung des Gehalts am Unteroxidationsprodukt Naphthochinon bei gleichbleibender Naphthalinkonzentration im Produkt.

## Patentansprüche

1. Verfahren zur Herstellung von Phthalsäureanhydrid durch Gasphasenoxidation von aromatischen Kohlenwasserstoffen, bei dem man einen Gasstrom, der mindestens einen aromatischen Kohlenwasserstoff und molekularen Sauerstoff umfasst, kontinuierlich über einen thermostatisierten Katalysator leitet, **dadurch gekennzeichnet, dass** man nach Inbetriebnahme des Katalysators die Zufuhr des mindestens einen aromatischen Kohlenwasserstoffs zum Katalysator für einen Zeitraum unterbricht und am Ende des Zeitraums die Zufuhr des mindestens einen aromatischen Kohlenwasserstoffs zum Katalysator wieder aufnimmt und die Temperatur des Wärmeträgermediums auf einen höheren Wert einstellt als vor der Unterbrechung der Zufuhr des aromatischen Kohlenwasserstoffs zum Katalysator.

2. Verfahren nach Anspruch 1, **dadurch gekennzeichnet, dass** die Länge des Zeitraums im Bereich von 30 Minuten bis 200 Tagen liegt.

3. Verfahren nach Anspruch 1 oder 2, **dadurch gekennzeichnet, dass** während des Zeitraums ein Kohlenwasserstoff-freier Gasstrom über den Katalysator geleitet wird.

4. Verfahren nach Anspruch 3, **dadurch gekennzeichnet, dass** der über den Katalysator geleitete Kohlenwasserstoff-freier Gasstrom zwischen 0 und 50 Vol.-% Sauerstoff enthält und ansonsten Luft, Stickstoff und/oder Edelgase umfasst.

5. Verfahren nach Anspruch 1 oder 2, **dadurch gekennzeichnet, dass** während des Zeitraums kein Gasstrom über den Katalysator geleitet wird.

6. Verfahren nach einem der Ansprüche 1 bis 5, **dadurch gekennzeichnet, dass** am Ende des Zeitraums die Beladung des Gasstroms mit aromatischem Kohlenwasserstoff auf einen niedrigeren Wert eingestellt wird als vor der Unterbrechung der Zufuhr des aromatischen Kohlenwasserstoffs zum Katalysator.

## Claims

1. A process for preparing phthalic anhydride by gas phase oxidation of aromatic hydrocarbons, in which a gas stream comprising at least one aromatic hydrocarbon and molecular oxygen is passed continuously over a thermostatted catalyst, which comprises interrupting the supply of the at least one aromatic hydrocarbon to the catalyst for a period after putting the catalyst on stream and resuming the supply of the at least one aromatic hydrocarbon to the catalyst at the end of the period and setting the temperature of the heat carrier medium to a higher value than before the interruption of the supply of the aromatic hydrocarbon to the catalyst.

2. The process according to claim 1, wherein the length of the period is in the range of 30 minutes to 200 days.

3. The process according to claim 1 or 2, wherein a hydrocarbon-free gas stream is passed over the catalyst during the period.

4. The process according to claim 3, wherein the hydrocarbon-free gas stream passed over the catalyst comprises between 0 and 50% by volume of oxygen and otherwise comprises air, nitrogen and/or noble gases.

5. The process according to claim 1 or 2, wherein no gas stream is passed over the catalyst during the period.

6. The process according to any of claims 1 to 5, wherein the loading of the gas stream with aromatic hydrocarbon is set to a lower value at the end of the period than before the interruption of the supply of the aromatic hydrocarbon to the catalyst.

## Revendications

1. Procédé pour la préparation d'anhydride de l'acide phtalique par oxydation en phase gazeuse d'hydrocarbures aromatiques dans lequel on guide un flux gazeux, qui comprend au moins un hydrocarbure aromatique et de l'oxygène moléculaire, en continu sur un catalyseur thermostaté, **caractérisé en ce qu'**après la mise en service du catalyseur, on interrompt l'alimentation dudit au moins un hydrocarbure aromatique sur le catalyseur pendant une certaine durée et on reprend de nouveau à la fin de la durée l'alimentation dudit au moins un hydrocarbure aromatique sur le catalyseur et on règle la température du milieu caloporteur à une valeur supérieure à celle avant l'interruption de l'alimentation de l'hydrocarbure aromatique sur le catalyseur.

2. Procédé selon la revendication 1, **caractérisé en ce que** la longueur de la durée se situe dans la plage de 30 minutes à 200 jours.

3. Procédé selon la revendication 1 ou 2, **caractérisé en ce que**, pendant la durée, un flux gazeux exempt d'hydrocarbures est guidé sur le catalyseur.

4. Procédé selon la revendication 3, **caractérisé en ce que** le flux gazeux exempt d'hydrocarbures guidé sur le catalyseur contient entre 0 et 50% en volume d'oxygène et pour le reste de l'air, de l'azote et/ou des gaz nobles.

5. Procédé selon la revendication 1 ou 2, **caractérisé en ce que**, pendant la durée, aucun flux gazeux n'est guidé sur le catalyseur.

6. Procédé selon l'une quelconque des revendications 1 à 5, **caractérisé en ce qu'**à la fin de la durée, la charge en hydrocarbure aromatique du flux gazeux est réglée à une valeur inférieure à celle avant l'interruption de l'alimentation de l'hydrocarbure aromatique sur le catalyseur.
